# EUROPEAN PATENT APPLICATION

(11) **EP 2 554 202 A1**
(43) Date of publication of application: **06.02.2013**
(21) Application number: 11762625.9
(22) Date of filing: 22.03.2011
(51) Int. Cl.: A61M 5/28, A61J 1/06, A61M 5/32

(54) **PREFILLED SYRINGE**

(30) Priority: 31.03.2010 JP 2010082587
(71) Applicant: Terumo Kabushiki Kaisha, Tokyo 151-0072 (JP)
(72) Inventor: OKAYAMA, Tomoki, Nakakoma-gun Yamanashi 409-3853 (JP); TACHIKAWA, Kouichi, Nakakoma-gun Yamanashi 409-3853 (JP); OGAWA, Junichi, Nakakoma-gun Yamanashi 409-3853 (JP)
(74) Representative: TBK
(86) International application number: PCT/JP2011/056732
(87) International publication number: WO 2011/122393

(57) **Abstract**

The present invention provides a prefilled syringe (1) which can reduce the amount of gas to be administrated together with medicinal solution (M). The prefilled syringe (1) of the present invention includes an outer tube (2) open at the opposite ends thereof, a medicinal solution vessel (5) in which medicinal solution (M) is filled, a sealing member (23), a pushing portion (22) for operating movement of the medicinal solution vessel (5), a needle tube (3), a needle hub (4), and a seal member (24). The medicinal solution vessel (5) slidably moves in the outer tube (2), is open at one end thereof and has a bottom portion (21 b) at the other end thereof. The sealing member (23) seals the one end of the medicinal solution vessel (5). The needle tube (3) has provided at one end thereof a first needle tip (9) for puncturing a living organism and has provided at the other end thereof a second needle tip for puncturing the sealing member (23). The needle hub (4) holds an intermediate portion of the needle tube (3) and is connected to one end of the outer tube (2) such that the second needle tip of the needle tube (3) is disposed in the outer tube (2). The seal member (24) seals between an inner circumferential face of the outer tube (2) and an outer circumferential face of the medicinal solution vessel (5). Further, the sealing member (23) has provided thereon a storing recessed portion (6a) for storing gas in the medicinal solution vessel (5).

## Description

### TECHNICAL FIELD

The present invention relates to a prefilled syringe wherein a medicinal solution is accommodated in a syringe in advance.

### BACKGROUND ART

In recent years, a prefilled syringe wherein a medicinal solution is filled in a syringe in advance has been and is used progressively. Such a prefilled syringe as just described eliminates the necessity to suck a medicinal solution into the syringe from a vial container upon medicinal administration and can reduce the time required for the administration.

Such a prefilled syringe as just described is disclosed, for example, in Patent Document 1. The prefilled syringe disclosed in Patent Document 1 includes a syringe main body in which a medicinal solution is filled, and a cap mounted on the syringe main body. The cap includes a rubber membrane provided therein for sealing a nozzle of the syringe main body.

The prefilled syringe configured in this manner can be used in two different methods. According to one of the methods, the cap is removed, and a luer connector or a hub of an injection needle is connected to the nozzle of the syringe main body. According to the other method, the rubber membrane of the cap is penetrated by a hollow double-headed needle. In both methods, a pusher provided on the syringe main body is pushed to discharge the medicinal solution from the nozzle or the injection needle (double-headed needle).

Meanwhile, another prefilled syringe is disclosed, for example, in Patent Document 2. In the prefilled syringe disclosed in Patent Document 2, a medicinal solution cartridge filled with a medicinal solution in advance is mounted on a syringe main body to communicate an injection needle fixed to the syringe main body and the medicinal solution cartridge with each other. Then, the medicinal solution cartridge is slidably moved in the inside of the syringe main body and a piston is inserted into the inside of the medicinal solution cartridge so that the medicinal solution is discharged from the injection needle.

### Prior Art Documents

### [Patent Documents]

Patent Document 1: Japanese Patent Laid-Open No. 2004-321826
Patent Document 2: Japanese Patent Laid-Open No. 2002-172166

### DISCLOSURE OF INVENTION

### Problem to be Solved by the Invention

However, in the prefilled syringes disclosed in Patent Documents 1 and 2, if gas remains in the syringe main body or the medicinal solution cartridge, then the gas is sometimes released from the injection needle (i.e. air venting is carried out) before the injection needle punctures a living organism. At this time, also the medicinal solution is discharged together with the gas, and this gives rise to a problem that part of the medicinal solution filled in the syringe main body or the medicinal solution cartridge is wasted.

Where a medicinal solution having a sedimentation property is used, since gas for intimately mixing the medicinal solution is filled in the medicinal solution vessel in advance, an operation for removing the air is required. However, the air for intimate mixing cannot sometimes be removed completely.

Further, it is investigated to administer vaccine to the upper layer part of the skin in which many immunocompetent cells exist to reduce the administration amount of the vaccine. It is to be noted that the upper layer part of the skin signifies the epidermis and the dermis of the skin. The administration amount of the vaccine to the upper layer part of the skin is approximately 50 to 300 µL, preferably approximately 100 µL. Accordingly, if the amount of the medicinal solution which is wasted is great, then the benefit that the amount of the antigen obtained by the administration of the vaccine to the upper layer part of the skin decreases is reduced.

Meanwhile, in subcutaneous administration or the like, gas for intimate mixing is sometimes administrated into the human body together with the medicinal solution. However, in order to moderate the burden imposed on the human body by administration of gas together with the medicinal solution, it is preferable to prevent the gas for intimate mixing from being administrated to the human body.

Taking the foregoing into consideration, it is an object of the present invention to provide a prefilled syringe wherein gas can be kept in a medicinal solution vessel in order to moderate the burden imposed on the human body.

### Means for Solving Problem

In order to solve the subject described above and achieve the object of the present invention, the prefilled syringe of the present invention includes an outer tube open at the opposite ends thereof, a medicinal solution vessel in which a medicinal solution is filled, a sealing member, a pushing portion, a needle tube, a needle hub, and a seal member.

The medicinal solution vessel slidably moves in the outer tube, is open at one end thereof and has a bottom portion at the other end thereof.

The sealing member seals the one end of the medicinal solution vessel.

The pushing portion is provided for operating movement of the medicinal solution vessel.

The needle tube has provided at one end thereof a first needle tip for puncturing a living organism and has provided at the other end thereof a second needle tip for puncturing the bottom portion of the medicinal solution vessel or the sealing member.

The needle hub holds an intermediate portion of the needle tube and is connected to one end of the outer tube such that the second needle tip of the needle tube is disposed in the outer tube.

The seal member seals between an inner circumferential face of the outer tube and an outer circumferential face of the medicinal solution vessel.

Further, at least one of the sealing member and the bottom portion of the medicinal solution vessel has provided thereon a storing portion for storing gas in the medicinal solution vessel.

In the prefilled syringe of the present invention, by causing the needle tube to puncture the bottom portion of the medicinal solution vessel or the sealing member and slidably moving the sealing member in the medicinal solution vessel, the medicinal solution is discharged from the needle tube. At this time, even if gas is accommodated in the medicinal solution vessel together with the medicinal solution, the gas is stored into the storing portion. Accordingly, the amount of gas to be administered together with the medicinal solution can be reduced.

### Effect of the Invention

With the prefilled syringe of the present invention, the amount of gas to be administered together with the medicinal solution can be reduced.

### BRIEF DESCRIPTION OF DRAWINGS

FIG. 1 is a sectional view showing a first embodiment of a prefilled syringe of the present invention.
FIG. 2 is a sectional view illustrating a state in which a needle tube in the first embodiment of the prefilled syringe of the present invention punctures a living organism.
FIG. 3 is a sectional view illustrating a state in which administration of a medicinal solution in the first embodiment of the prefilled syringe of the present invention is completed.
FIG. 4A is a partial sectional view showing a second embodiment of a prefilled syringe of the present invention, and FIG. 4B is a sectional view illustrating a state in which administration of a medicinal solution by the second embodiment of the prefilled syringe of the present invention is completed.
FIG. 5A is a partial sectional view showing a third embodiment of a prefilled syringe of the present invention, and FIG. 5B is a sectional view illustrating a state in which administration of a medicinal solution by the third embodiment of the prefilled syringe of the present invention is completed.
FIG. 6A is a partial sectional view showing a fourth embodiment of a prefilled syringe of the present invention, and FIG. 6B is a sectional view illustrating a state in which administration of a medicinal solution by the fourth embodiment of the prefilled syringe of the present invention is completed.
FIG. 7 is a partial sectional view showing a fifth embodiment of a prefilled syringe of the present invention.
FIG. 8 is a sectional view illustrating a state in which a needle tube in the fifth embodiment of the prefilled syringe of the present invention punctures a living organism.
FIG. 9 is a sectional view illustrating a state in which administration of a medicinal solution in the fifth embodiment of the prefilled syringe of the present invention is completed.

### MODE FOR CARRYING OUT THE INVENTION

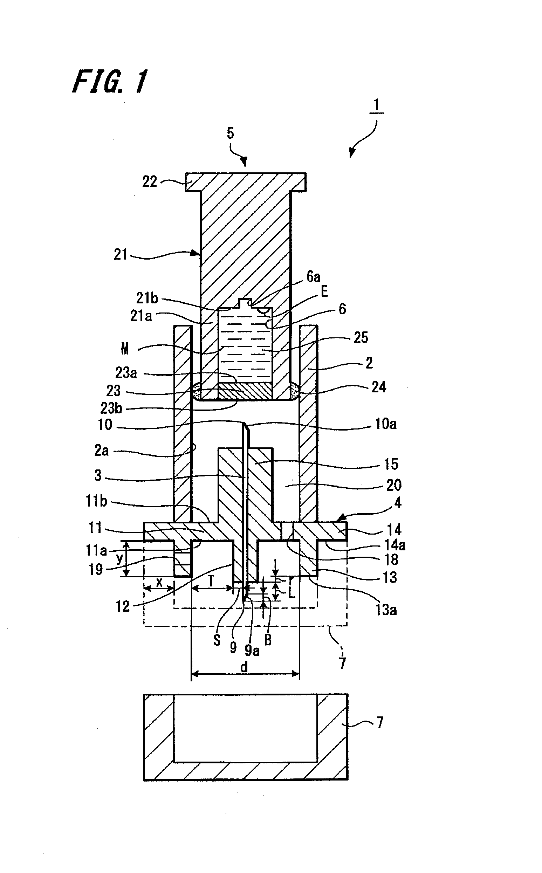

In the following, embodiments of the prefilled syringe of the present invention are described with reference to FIGS. 1 to 9. It is to be noted that, in the figures, like members are denoted by like letters or numerals. Further, the present invention is not limited to the following embodiments.

It is to be noted that the description is given in the following order.
1. First Embodiment
1-1. Configuration of the Prefilled Syringe
1-2. Method of Use of the Prefilled Syringe
2. Second Embodiment
3. Third Embodiment
4. Fourth Embodiment
5. Fifth Embodiment

### <1. First Embodiment>

### 1-1. Example of the Configuration of the Prefilled Syringe

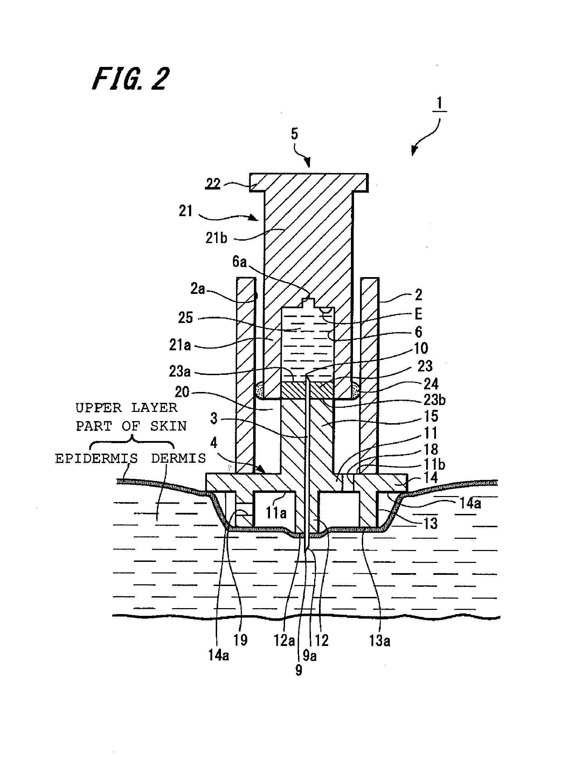

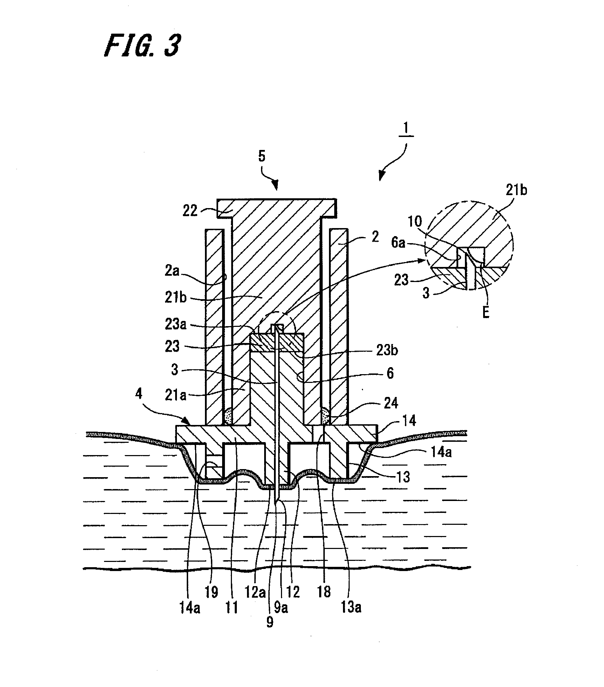

First, a prefilled syringe according to a first embodiment of the present invention (hereinafter referred to as "present embodiment") is described with reference to FIGS. 1 to 3. FIG. 1 is a sectional view showing the prefilled syringe of the first embodiment, and FIG. 2 is a sectional view illustrating a state in which a needle tube of the prefilled syringe of the first embodiment punctures the skin. FIG. 3 is a sectional view illustrating a state in which administration of medicinal solution is completed.

As shown in FIG. 1, the prefilled syringe 1 stores a medicinal solution M to be administrated into a living organism in advance therein. This prefilled syringe 1 includes an outer tube 2, a hollow needle tube 3 having a needle hole, a needle hub 4 for holding the needle tube 3, a medicinal solution vessel 5 having a medicinal solution storage portion 6, a sealing member 23 for sealing an opening of the medicinal solution storage portion 6, and a cap 7.

Although, in the present embodiment, various vaccines for preventing various infections such as, for example, influenza are applicable as the medicinal solution M to be accommodated in the prefilled syringe 1 in advance, the medicinal solution M is not limited to the vaccines. It is to be noted that, in addition to the vaccines, for example, carbohydrate injection solutions of glucose and so forth, injection solutions for electrolyte correction of sodium chloride, potassium lactate and so forth, vitamins, antibiotic injection solutions, radiopaque agents, steroid drugs, proteolytic enzyme inhibitors, fat emulsions, anti-cancer agents, anesthetics, stimulants, narcotics, heparin calcium, antibody drugs and so forth are applicable.

### [Outer Tube]

First, the outer tube 2 is described.

The outer tube 2 is formed in a cylindrical shape having a cylindrical hole 2a. The outer tube 2 is open at the opposite ends thereof in the axial direction.

As the material for the outer tube 2, various resins such as, for example, polyvinyl chloride, polyethylene, polypropylene, cyclic polyolefins, polystyrene, poly(4-methyl pentene-1), polycarbonates, acrylic resins, acrylonitrile-butadiene-styrene copolymer, polyesters such as polyethylene terephthalate, butadiene-styrene copolymer, and polyamides (for example, nylon 6, nylon 6,6, nylon 6,10 or nylon 12) are applicable. It is preferable to use, among them, such resins as polypropylene, cyclic polyolefins, polyesters and poly(4-methyl pentene-1) in that they are easy to mold. It is to be noted that preferably the material of the outer tube 2 is substantially transparent in order that the visibility of the inside is assured.

### [Needle Tube]

Next, the needle tube 3 is described.

As the needle tube 3, a needle tube of a size of the 22 to 33 gauges (outer diameter: 0.2 to 0.7 mm) in accordance with the standard (ISO 9626: 1991 /Amd. 1 :2001 (E)) for needle tubes for medical use of the ISO can be used. Preferably, a needle tube of the 26 gauge to 33 gauge can be used, and more preferably, a needle tube of the 30 to 33 gauge can be used.

A first needle tip 9 to puncture the living organism is provided at one end of the needle tube 3, and a second needle tip 10 to puncture the sealing member 23 is provided at the other end of the needle tube 3. The first needle tip 9 has a blade surface 9a. The length of the blade surface 9a in a direction in which the needle tube 3 extends (the length is hereinafter referred to as "bevel length B") may be not more than 1.4 mm (adult) which is an optimum thickness of the upper layer part of the skin hereinafter described, and may be not less than approximately 0.5 mm which is the bevel length when a short bevel is formed on a needle tube of the 33 gauge. In other words, the bevel length B is preferably set within the range of 0.5 to 1.4 mm.

More preferably, the bevel length B is smaller than 0.9 mm (child) which is an optimum thickness of the upper layer part of the skin. In other words, it is more preferable to set the bevel length B within the range of 0.5 to 0.9 mm. It is to be noted that the "short bevel" indicates a blade surface having an angle of 18 to 25 degrees with respect to the longitudinal direction of the needle, which is used generally in needles for injection.

The second needle tip 10 has a blade surface 10a. Although the length of the blade surface 10a in the direction in which the needle tube 3 extends can be set arbitrarily, it can be set to an equal length to that of the blade surface 9a of the first needle tip 9.

As the material for the needle tube 3, for example, stainless steel, aluminum, aluminum alloy, titanium, titanium alloy and other metals can be used. Further, as the needle tube 3, a straight needle and a tapering needle which has a tapering structure at least at part thereof can be used. The tapering needle may have such a tapering structure at an intermediate portion thereof that the outer diameter thereof on the second needle tip 10 side is greater than the outer diameter thereof on the first needle tip 9 side. It is to be noted that, in this instance, the first needle tip 9 and the second needle tip 10 are different in shape from each other.

### [Needle Hub]

Next, the needle hub 4 is described.

The needle hub 4 holds an intermediate portion of the needle tube 3. The needle hub 4 includes a hub main body 11 of a substantially disk shape, an adjustment portion 12, a stabilization portion 13, a guide portion 14 serving as a pressure indication portion, and an engaging portion 15. The needle hub 4 is connected to one end of the outer tube 2 in a state it holds the needle tube 3 and closes up an opening on the one end side of the outer tube 2. Consequently, the second needle tip 10 of the needle tube 3 held by the needle hub 4 is disposed in an internal space 20 formed by the cylindrical hole 2a of the outer tube 2 and the needle hub 4.

The adjustment portion 12 and the stabilization portion 13 are provided on one end side (one flat face) of the hub main body 11 while the engaging portion 15 is provided on the other end side (other flat face) of the hub main body 11. As the material for the needle hub 4, though not limited specifically, for example, materials similar to those of the outer tube 2 described hereinabove can be used. Further, the needle hub 4 may be formed integrally with the outer tube 2.

Further, a first vent hole 18 is provided in the hub main body 11 such that it extends through the hub main body 11 from one end side to the other end side. The internal space 20 defined by the cylindrical hole 2a of the outer tube 2 and the needle hub 4 and the outside of the prefilled syringe 1 are communicated with each other by the first vent hole 18. It is to be noted that a filter may be provided in the first vent hole 18 in order to raise the maintenance of a sterilized state of the internal space 20 defined by the cylindrical hole 2a of the outer tube 2 and the needle hub 4.

While, in the present embodiment, an example wherein one first vent hole 18 is provided is described, the number of such first vent holes 18 is not limited to this, but a plurality of first vent holes 18 may be formed in the hub main body 11.

Next, the adjustment portion 12 is described.

The adjustment portion 12 is provided at a central portion of one end face (one flat face) 1 1 a of the hub main body 11 and formed as a convex portion which protrudes in the axial direction of the hub main body 11. The axis of the adjustment portion 12 coincides with the axis of the hub main body 11. The needle tube 3 extends through the adjustment portion 12. Further, an end face of the adjustment portion 12 serves as a needle protruding face 12a from which the first needle tip 9 side of the needle tube 3 protrudes.

The needle protruding face 12a is formed as a flat face perpendicular to the axial direction of the needle tube 3. This needle protruding face 12a defines the depth by which the needle tube 3 punctures after it is brought into contact with the surface of the skin when the needle tube 3 punctures the upper layer part of the skin. In other words, the depth by which the needle tube 3 punctures the upper layer part of the skin is determined by the length of the needle tube 3 protruding from the needle protruding face 12a (the length is hereinafter referred to as "protruding length L").

The thickness of the upper layer part of the skin corresponds to the depth from the surface of the skin to the dermis layer and is included in a range of substantially 0.5 to 3.0 mm. Therefore, the protruding length L of the needle tube 3 can be set within the range from 0.5 to 3.0 mm.

Incidentally, although a vaccine is generally administrated to the brachial region, where administration to the upper layer part of the skin is taken into consideration, it is considered preferable that a vaccine is administrated to a peripheral region of a shoulder in which the skin is thick, particularly to a deltoid region. Therefore, the thickness of the upper layer part of the skin at the deltoid was measured on 19 children and 31 adults. This measurement was carried out by sonography of the upper layer part of the skin having a high ultrasonic reflectivity using an ultrasonic measuring instrument (NP60R-UBM, high resolution echoscope for small animals, Nepa Gene Co., Ltd.). It is to be noted that, since the measurement values exhibited a logarithmic normal distribution, the range of MEAN ± 2SD was determined by geometric mean.

As a result, the thickness of the upper layer part of the skin at the deltoid of the children was 0.9 to 1.6 mm. Meanwhile, the thickness of the upper layer part of the skin at the deltoid of the adults was 1.4 to 2.6 mm at a distal portion, 1.4 to 2.5 mm at a middle portion and 1.5 to 2.5 mm at a proximal portion. From the foregoing, it was confirmed that the thickness of the upper layer part of the skin at the deltoid of the children was not less than 0.9 mm and that of the adults was not less than 1.4 mm. Accordingly, in injection to the upper layer part of the skin at the deltoid, preferably the protruding length L of the needle tube 3 is set within the range of 0.9 to 1.4 mm.

By setting the protruding length L in this manner; it becomes possible to position the blade surface 9a of the first needle tip 9 at the upper layer part of the skin with certainty. As a result, the needle hole (medicinal solution discharge port) which is open to the blade surface 9a can be positioned at the upper layer part of the skin at any position in the blade surface 9a. It is to be noted that, even if the medicinal solution discharge port is positioned at the upper layer part of the skin, if the first needle tip 9 sticks too deeply into the upper layer part of the skin, then the medicinal solution M flows out under the subcutaneous part from between the side face of the end portion of the first needle tip 9 and the incised skin. Therefore, it is significant that the blade surface 9a is positioned at the upper layer part of the skin with certainty.

It is to be noted that, in the case of a thicker needle tube than the 26 gauge, it is difficult to make the bevel length B not more than 1.0 mm. Accordingly, in order to set the protruding length L of the first needle tip 9 of the needle tube 3 to a length within the preferable range (0.9 to 1.4 mm), it is preferable to use a needle tube thinner than the 26 gauge.

The needle protruding face 12a is formed such that the distance S from a circumferential edge thereof to the circumferential face of the needle tube 3 is not more than 1.4 mm and preferably within a range of 0.3 to 1.4 mm. The distance S from the circumferential edge of the needle protruding face 12a to the circumferential edge of the needle tube 3 is set taking it into consideration that pressure is applied to a water blister formed by administration of the medicinal solution to the upper layer part of the skin. In particular, the needle protruding face 12a is set to a size which is sufficiently smaller than the water blister formed at the upper layer part of the skin and does not prevent formation of a water blister. As a result, it can be prevented that the needle protruding face 12a presses the skin around the needle tube 3 and the administrated medicinal solution leaks.

Next, the stabilization portion 13 is described.

The stabilization portion 13 is provided on the one end face 11 a of the hub main body 11. The stabilization portion 13 is formed in a continuous tubular shape on a circumferential edge portion of the one end face 11a. The first needle tip 9 of the needle tube 3 and the adjustment portion 12 are disposed in the cylindrical hole of the stabilization portion 13. In other words, the stabilization portion 13 is formed as a tube which covers the circumference of the adjustment portion 12 through which the needle tube 3 extends.

Further, if the first needle tip 9 of the needle tube 3 punctures a living organism as shown in FIG. 2, then the needle protruding face 12a as well as an end face 13a of the stabilization portion 13 is brought into contact with the surface of the skin. At this time, since the end face 13a of the stabilization portion 13 contacts with the skin, the posture of the prefilled syringe 1 is stabilized and the needle tube 3 can be kept in a posture substantially perpendicular to the skin.

It is to be noted that, even if the end face 13a of the stabilization portion 13 is positioned on the same plane as the needle protruding face 12a or positioned to the first needle tip 9 side of the needle tube 3 with respect to the needle protruding face 12a, the needle tube 3 can be kept in a posture substantially perpendicular to the skin. It is to be noted that, if swelling of the skin when the stabilization portion 13 is pressed against the skin is taken into consideration, then the distance r in the axial direction between the end face 13a of the stabilization portion 13 and the needle protruding face 12a is preferably set to not more than 1.3 mm.

Further, the inner diameter d of the stabilization portion 13 is set to a value equal to or higher than the value of the diameter of a water blister formed on the skin. In particular, the inner diameter d of the stabilization portion 13 is set such that the distance T from an inner wall face of the stabilization portion 13 to a circumferential edge of the needle protruding face 12a falls within the range of 4 mm to 15 mm. By this setting, obstruction of formation of the water blister by application of a pressure to a water blister from the inner wall face of the stabilization portion 13 can be prevented.

The distance T from an inner wall face of the stabilization portion 13 to a circumferential edge of the needle protruding face 12a does not specifically have an upper limit only if it is not less than 4 mm. However, if the distance T is increased, then since the outer diameter of the stabilization portion 13 increases, in the case where the needle tube 3 punctures a thin arm as in the case of a child, it is difficult to contact the entire end face 13a of the stabilization portion 13 with the skin. Therefore, preferably the distance T is defined such that the maximum value thereof is 15 mm taking the thinness of an arm of a child into consideration.

Meanwhile, if the distance S from a circumferential edge of the needle protruding face 12a to a circumferential face of the needle tube 3 is not less than 0.3 mm, then the adjustment portion 12 does not advance into the skin. Accordingly, if the distance T (not less than 4 mm) from the inner wall face of the stabilization portion 13 to the circumferential edge of the needle protruding face 12a and the diameter (approximately 0.3 mm) of the needle protruding face 12a are taken into consideration, then the inner diameter d of the stabilization portion 13 can be set to not less than 9 mm.

Further, a second vent hole 19 is formed in the stabilization portion 13 such that it extends from the outer circumferential face to the inner circumferential face of the stabilization portion 13 through the stabilization portion 13. By providing the second vent hole 19 in the stabilization portion 13, when the stabilization portion 13 is brought into contact with the skin as shown in FIG. 2, the space defined by the stabilization portion 13 and the skin and the space on the outer side of the prefilled syringe 1 can be communicated with each other. Thus, the first vent hole 18 and the second vent hole 19 configure an air communicating means for communicating the air between the internal space 20 and the outer side of the prefilled syringe 1.

It is to be noted that the shape of the stabilization portion 13 is not limited to a cylindrical shape, but the stabilization portion 13 may be formed in a polygonal tubular shape such as, for example, a tetragonal prism or a hexagonal prism having a cylindrical hole at the center thereof.

Next, the guide portion 14 which is a pressure indication portion is described.

The guide portion 14 is provided on a side face portion of the hub main body 11. The guide portion 14 is formed as a ring-shaped flange protruding in a radially outward direction of the hub main body 11 from the side face portion of the hub main body 11. The guide portion 14 protrudes substantially perpendicularly to the outer circumferential face of the stabilization portion 13.

Further, the guide portion 14 has a contact face 14a for contacting with the skin. The contact face 14a is a flat face extending substantially in parallel to the end face 13a of the stabilization portion 13. By pressing the stabilization portion 13 until the contact face 14a of the guide portion 14 is brought into contact with the skin, the force of the stabilization portion 13 and the needle tube 3 pressing the skin can always be assured with a value equal to or higher than a predetermined value. Consequently, a portion of the needle tube 3 which protrudes from the needle protruding face 12a (corresponding to the protruding length L) punctures the skin with certainty.

The distance from the contact face 14a of the guide portion 14 to the end face 13a of the stabilization portion 13 is set such that the needle tube 3 can puncture the skin with suitable pressing force applied to the skin from the stabilization portion 13 and the needle tube 3 (also refer to FIG. 3). In the following description, this length is referred to as "guide portion height y."

It is to be noted that appropriate pressing force of the needle tube 3 and the stabilization portion 13 is, for example, 3 to 20 N. As a result, the pressing force to the skin from the needle tube 3 and the stabilization portion 13 can be guided to the user by the guide portion 14 and the first needle tip 9 and the blade surface 9a of the needle tube 3 can be positioned with certainty at the upper layer part of the skin. Therefore, an effect that the sense of security can be provided to the user is achieved.

In particular, in the case where the inner diameter d of the stabilization portion 13 is within a range from 11 to 14 mm, the guide portion height y is set suitably based on the length x (hereinafter referred to as "guide portion length x") from a protruding end face of the guide portion 14 to an outer circumferential face of the stabilization portion 13. For example, in the case where the inner diameter d of the stabilization portion 13 is 12 mm and the guide portion length x is 3.0 mm, the guide portion height y is set within a range from 2.3 to 6.6 mm.

Next, the engaging portion 15 is described.

The engaging portion 15 is provided at a central portion of the other end face (the other flat face) 1 1 b opposing to the one end face 11 a of the hub main body 11. This engaging portion 15 is configured as a projection which protrudes in a substantially column shape in the axial direction of the hub main body 11. The needle tube 3 extends through the engaging portion 15, and the second needle tip 10 of the needle tube 3 protrudes from the end face of the engaging portion 15.

In the present embodiment, the axis of the needle tube 3 and the axis of the adjustment portion 12 and the engaging portion 15 coincide with each other. However, even if the axis of the needle tube 3 and the axis of the engaging portion 15 do not coincide with each other, the intended object can be achieved.

This engaging portion 15 is disposed in the internal space 20 defined by the cylindrical hole 2a of the outer tube 2 and the needle hub 4 when the opening on one end side of the outer tube 2 is closed up with the needle hub 4.

### [Medicinal solution Vessel]

Next, the medicinal solution vessel 5 is described.

The medicinal solution vessel 5 is inserted in the cylindrical hole 2a of the outer tube 2 and serves also as a pusher for moving the sealing member 23. This medicinal solution vessel 5 has a vessel main body 21 of a substantially cylindrical shape having the medicinal solution storage portion 6, and a pushing portion 22 provided at one end of the vessel main body 21. The diameter of the vessel main body 21 is formed smaller than the diameter of the cylindrical hole 2a of the outer tube 2.

The pushing portion 22 is formed in the shape of a ring which protrudes in a radially outward direction of the vessel main body 21 from a side face portion of the vessel main body 21 and has an outer diameter formed greater than the diameter of the cylindrical hole 2a of the outer tube 2. If the pushing portion 22 is pushed by the user, then the vessel main body 21 moves in the cylindrical hole 2a of the outer tube 2 along the axial direction of the cylindrical hole 2a.

Next, the medicinal solution storage portion 6 is described.

The medicinal solution storage portion 6 is provided at the other end portion of the vessel main body 21 in the axial direction. The medicinal solution storage portion 6 is formed by providing a recessed portion on the other end face of the vessel main body 21 in the axial direction. In particular, the vessel main body 21 is configured from a tubular portion 21 a open at the opposite ends thereof, and a bottom portion 21 b of a cylindrical shape which closes up one of the openings of the tubular portion 21 a, and forms the medicinal solution storage portion 6. A storing recessed portion 6a serving as a storage portion is provided on a face of the bottom portion 21 b which forms the medicinal solution storage portion 6 (bottom face of the medicinal solution storage portion 6).

The storing recessed portion 6a is provided at a position opposing to the second needle tip 10 of the needle tube 3 with the sealing member 23 interposed therebetween. The cross section of the storing recessed portion 6a in a direction perpendicular to the axial direction of the vessel main body 21 is formed greater than the outer diameter of the needle tube 3. Further, the depth of the storing recessed portion 6a is set equal to or greater than the length by which the second needle tip 10 of the needle tube 3 protrudes from the sealing member 23. In a state in which administration of the medicinal solution is completed, the second needle tip 10 side of the needle tube 3 is disposed in the storing recessed portion 6a.

The medicinal solution M is accommodated in the medicinal solution storage portion 6. The diameter of the opening of the medicinal solution storage portion 6 is set substantially equal to or a little greater than the diameter of the engaging portion 15. Further, the sealing member 23 is attached to the medicinal solution storage portion 6 such that it closes up the opening of the medicinal solution storage portion 6. Therefore, the medicinal solution M is sealed in a medicinal solution space 25 defined by the medicinal solution storage portion 6 and the sealing member 23. The medicinal solution space 25 is an enclosed space sealed airtight by the sealing member 23 such that a sterilized state is maintained.

It is to be noted that, although the amount of the medicinal solution M accommodated in the medicinal solution space 25 (the volume of the medicinal solution space 25) is not restricted specifically, preferably it is, for example, approximately 0.02 to 2.0 mL, and more preferably, it is approximately 0.05 to 0.8 mL. In other words, the prefilled syringe 1 is suitable particularly in the case where such a small amount of medicinal solution is to be administrated.

### [Sealing Member]

Next, the sealing member 23 is described.

The sealing member 23 is slidably inserted in the tubular portion 21 a of the vessel main body 21 and closes up the medicinal solution space 25 liquid-tightly. The sealing member 23 has a contacting face 23b which can contact in surface contact with the bottom portion 21 b of the vessel main body 21, and an abutting face 23b for abutting with the engaging portion 15 of the needle hub 4. Further, the sealing member 23 moves together with the vessel main body 21, whereupon it is penetrated by the second needle tip 10 side of the needle tube 3.

Further, although the material of the sealing member 23 is not specifically limited, the sealing member 23 is preferably configured from an elastic material in order to assure a good liquid-tightness with the medicinal solution storage portion 6. For example, various rubber materials of natural rubber, butyl rubber, isoprene rubber, isobutylene rubber, butadiene rubber, styrene-butadiene rubber, silicone number and so forth, various thermoplastic elastomers of polyurethane-based, polyester-based, polyamide-based, olefin-based and styrene-based types elastomers and the like, or mixtures of them and so forth can be used as the elastic materials.

It is to be noted that the sealing member 23 may be formed such that at least an outer circumferential portion thereof is configured from an elastic material as described above and may have a configuration wherein it has a core portion (not shown) configured, for example, from a resin material and the elastic material described above is disposed in such a manner as to cover an outer periphery of the core portion.

Although the material of the medicinal solution vessel 5 having such a configuration as described above is not limited specifically, for example, materials similar to those of the outer tube 2 described hereinabove can be used. It is to be noted that preferably the material of the medicinal solution vessel 5 is substantially transparent in order that the visibility of the inside can be assured. Further, graduations may be formed on the outer circumferential face of the medicinal solution storage portion 6 of the medicinal solution vessel 5. Based on the graduations, the amount of the medicinal solution M accommodated in the medicinal solution storage portion 6 can be grasped.

Further, a seal member 24 is attached to an outer circumferential face of the vessel main body 21 on the medicinal solution storage portion 6 side. The seal member 24 is an O-snap ring and is provided continuously along a circumferential direction on the outer circumferential face of the vessel main body 21. It is to be noted that the seal member 24 may be formed integrally with the medicinal solution vessel 5 by two-color molding.

When the medicinal solution vessel 5 is inserted into the cylindrical hole 2a of the outer tube 2, the seal member 24 closely contacts with the outer circumferential face of the vessel main body 21 and an inner wall of the cylindrical hole 2a. Therefore, the cylindrical hole 2a of the outer tube 2 and the vessel main body 21 are sealed fully therebetween. As a result, the internal space 20 defined by the cylindrical hole 2a of the outer tube 2 and the hub main body 11 of the needle hub 4 is sealed airtight by the seal member 24. Further, since the seal member 24 closely contacts with the outer circumferential face of the vessel main body 21 and the inner wall of the cylindrical hole 2a, the vessel main body 21 can be held stably at an intermediate portion of the cylindrical hole 2a of the outer tube 2.

Although the material of the seal member 24 is not specifically limited, materials similar to those of the sealing member 23 can be used.

### [Cap]

### Next, the cap 7 is described.

The cap 7 is a member for sealing the space which includes the first needle tip 9 of the needle tube 3 and surrounds the adjustment portion 12 and the stabilization portion 13 of the needle hub 4. The cap 7 is removably formed on the stabilization portion 13 of the needle hub 4.

This cap 7 is formed as a bottomed tube having a bottom portion on the tip end side thereof, in the present embodiment, as a bottomed cylinder. In an unused state of the prefilled syringe 1, the cap 7 is mounted on the stabilization portion 13 to enclose the second vent hole 19 and the inside of the cap 7. By the structure, the sterilized state of the internal space 20 and the inside of the cap 7 is maintained.

When the prefilled syringe 1 is to be used, the cap 7 is removed from the needle hub 4 and the sealing of the first needle tip 9 of the needle tube 3 is canceled. Further, the second vent hole 19 of the needle hub 4 is opened. Consequently, in a state in which the stabilization portion 13 contacts with the skin as illustrated in FIG. 2, the space defined by the stabilization portion 13 and the skin and the internal space 20 can be opened to the outside by the first vent hole 18 and the second vent hole 19.

Although the material of the cap 7 is not limited specifically, for example, materials similar to those of the outer tube 2 or those of the medicinal solution vessel 5 described hereinabove may be used.

### 1-2. Method of Use of the Prefilled Syringe

Next, a method of use of the prefilled syringe 1 of the present embodiment is described with reference to FIGS. 1 to 3.

First, the cap 7 is removed from the stabilization portion 13 of the needle hub 4 as illustrated in FIG. 1. Consequently, the sealing of the first needle tip 9 of the needle tube 3 is canceled and the second vent hole 19 of the needle hub 4 is opened. Then, the end face 13a of the stabilization portion 13 is opposed to the skin. Consequently, the first needle tip 9 of the needle tube 3 is opposed to the skin to be punctured. Then, the prefilled syringe 1 is moved substantially perpendicularly to the skin so that the needle tube 3 punctures the skin and the end face 13a of the stabilization portion 13 is pressed against the skin, as shown in FIG. 2.

Here, the needle protruding face 12a of the adjustment portion 12 and the end face 13a of the stabilization portion 13 are positioned on the same plane. Consequently, the needle protruding face 12a of the adjustment portion 12 can be placed into contact with the skin to deform the skin so as to be flat, and the first needle tip 9 of the needle tube 3 can puncture the skin by the protruding length L.

Then, the stabilization portion 13 is pressed until the contact face 14a of the guide portion 14 is brought into contact with the skin. Here, the guide portion height y is set such that the needle tube 3 and the stabilization portion 13 can puncture the skin with appropriate pressing force. Therefore, the force of pressing the skin becomes a predetermined value by the stabilization portion 13. Accordingly, the pressing force of the stabilization portion 13 can be guided to the user and the stabilization portion 13 can be pressed against the skin with appropriate pressing force, and the first needle tip 9 and the blade surface 9a of the needle tube 3 can be positioned with certainty in the upper layer part of the skin.

Since the guide portion 14 becomes an indicator for guiding the pressing force of the stabilization portion 13 in this manner, the first needle tip 9 of the needle tube 3 can be positioned in the upper layer part of the skin with certainty. Thus, the medicinal solution can be administrated with certainty into the upper layer part of the skin and the sense of security of the user can be enhanced.

Further, as the stabilization portion 13 contacts with the skin, the needle tube 3 can be stabilized so that the needle tube 3 straightforwardly punctures the skin. Therefore, deflection which may occur with the needle tube 3 can be prevented and stabilized administration of the medicinal solution can be achieved.

In the case of a needle having a very small protrusion length, for example, of approximately 0.5 mm, even if the first needle tip 9 is contacted with the skin, it may not sometimes pierce into the skin. However, if the stabilization portion 13 is pressed against the skin to push down the skin in the vertical direction, then a state in which the skin on the inner side of the stabilization portion 13 is pulled and tension is applied to the skin is entered. Therefore, since the skin becomes less likely to escape with respect to the first needle tip 9 of the needle tube 3, the stabilization portion 13 has an effect also that the first needle tip 9 becomes easier to pierce into the skin.

Further, since the protruding length L is set within the range of 0.5 to 3.0 mm, the first needle tip 9 and the blade surface 9a of the needle tube 3 are positioned in the upper layer part of the skin with certainty. The adjustment portion 12 closely contacts with and is fixed to a circumference of the needle tube 3 such that a gap does not appear between a portion of the needle tube 3 which extends through the adjustment portion 12 and the adjustment portion 12.

Therefore, if the needle protruding face 12a of the adjustment portion 12 is brought into contact with the skin, then the skin around the needle tube 3 can be deformed into a flat state. As a result, the needle tube 3 can puncture the skin by the protruding length L, and the first needle tip 9 of the needle tube 3 can be positioned in the upper layer part of the skin with certainty.

Then, the user would press the pushing portion 22 side of the medicinal solution vessel 5 to slidably move the vessel main body 21, in which the medicinal solution M is filled, along the axial direction of the outer tube 2. Consequently, the abutting face 23b of the sealing member 23, which moves together with the vessel main body 21, is brought into abutment with the engaging portion 15 of the needle hub 4 (refer to FIG. 2). At this time, the second needle tip 10 of the needle tube 3 punctures the sealing member 23 and penetrates the sealing member 23. Consequently, the communication of the medicinal solution M accommodated in the medicinal solution storage portion 6 with the needle tube 3 is completed.

The first vent hole 18 and the second vent hole 19 are provided in the needle hub 4. When the medicinal solution vessel 5 is operated to move, the air in the internal space 20 is discharged to the outside through the first vent hole 18 and the second vent hole 19. Therefore, the pressure in the space defined by the internal space 20 and stabilization portion 13 and the skin can be prevented from rising. Consequently, the moving operation of the medicinal solution vessel 5 can be carried out readily and smoothly. Further, after administration of the medicinal solution M ends, even if the hand is removed from the medicinal solution vessel 5, the medicinal solution vessel 5 can be prevented from moving to the other end portion side of the outer tube 2.

Then, if the pushing portion 22 side of the medicinal solution vessel 5 is pushed toward the needle hub 4 side of the outer tube 2 as shown in FIG. 3, then the movement of the sealing member 23 is stopped by the engaging portion 15 while only the medicinal solution vessel 5 slid in the axial direction along the inner face of the outer tube 2. As a result, the sealing member 23 is pushed into the medicinal solution storage portion 6 and the medicinal solution M in the medicinal solution storage portion 6 is discharged from the first needle tip 9 into a living organism past the second needle tip 10 of the needle tube 3.

Thereafter, the contact face 23a of the sealing member 23 is brought into surface contact with the bottom portion 21 b of the medicinal solution vessel 5, and the administration of the medicinal solution M using the prefilled syringe 1 of the present embodiment is completed. At this time, even if gas E is accommodated in the medicinal solution storage portion 6 together with the medicinal solution M, the gas E partly or entirely stays in the storing recessed portion 6a. Accordingly, the amount of the gas to be administrated to the living organism past the second needle tip 10 of the needle tube 3 together with the medicinal solution M can be reduced.

Further, the second needle tip 10 side of the needle tube 3 protruding from the contact face 23a of the sealing member 23 is disposed in the storing recessed portion 6a. In particular, the storing recessed portion 6a functions as a cutout portion for avoiding interference between the second needle tip 10 side of the needle tube 3 and the bottom portion 21 b of the medicinal solution vessel 5, and the second needle tip 10 of the needle tube 3 and the bottom portion 21 b of the medicinal solution vessel 5 do not interfere with each other. As a result, the second needle tip 10 does not scrape the medicinal solution vessel 5 and a broken piece of the medicinal solution vessel 5 can be prevented from mixing into the medicinal solution M. Further, it can be prevented that the second needle tip 10 side of the needle tube 3 is deformed to block the needle hole, and a prescribed amount of the medicinal solution M can be discharged into the living organism with certainty from the first needle tip 9.

Further, since the needle protruding face 12a of the adjustment section 12 and the inner diameter d of the stabilization portion 13 are set to appropriate sizes, the administrated medicinal solution can be prevented from leaking to the outside of the living organism and can be administrated into the upper layer part of the skin with certainty.

### <2. Second Embodiment>

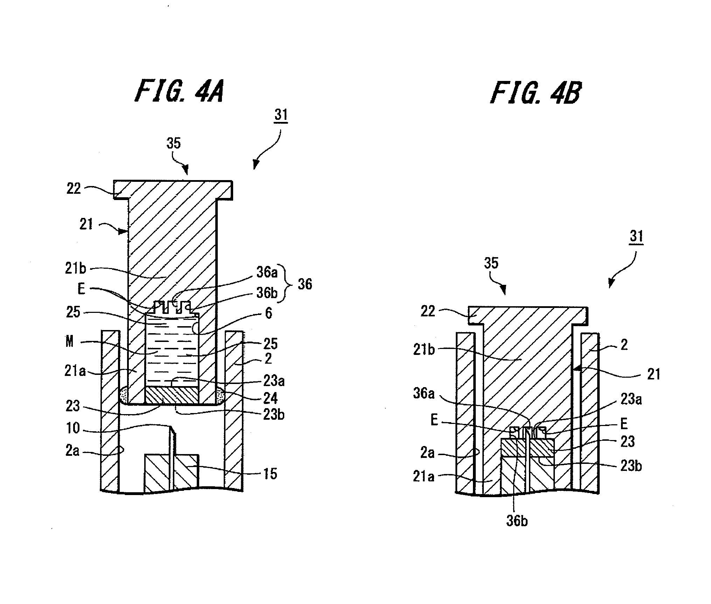

Next, a second embodiment of a prefilled syringe of the present invention is described with reference to FIG. 4.

FIG. 4A is a sectional view showing the prefilled syringe of the second embodiment, and FIG. 4B is a sectional view illustrating a state in which administration of a medicinal solution is completed.

The prefilled syringe 31 according to the present second embodiment is different from the prefilled syringe 1 according to the first embodiment in the storing recessed portion provided on a medicinal solution vessel. Therefore, description here is given of the medicinal solution vessel, and elements common to the prefilled syringe 1 are denoted by like letters or numerals and overlapping description of them is omitted.

As shown in FIGS. 4A and 4B, the medicinal solution vessel 35 has a vessel main body 21 of a substantially cylindrical shape having a medicinal solution storage portion 6, and a pressing portion 22 provided at one end of the vessel main body 21. Further, a storing recessed portion 36 which is a storing portion is provided on a face of the bottom portion 21 b of the vessel main body 21 on which the medicinal solution storage portion 6 is formed (on the bottom face of the medicinal solution storage portion 6).

The storing recessed portion 36 is configured from a storing recessed portion 36a provided at a central portion of the vessel main body 21, and a plurality of storing recessed portions 36b provided around the storing recessed portion 36a. The storing recessed portion 36a is opposed to the second needle tip 10 of the needle tube 3 with the sealing member 23 interposed therebetween. The sectional area of the storing recessed portion 36a in a direction perpendicular to the axial direction of the vessel main body 21 is formed greater than the outer diameter of the needle tube 3. Further, the depth of the storing recessed portion 36a is set equal to or longer than the length by which the second needle tip 10 side of the needle tube 3 protrudes from the sealing member 23. In a state in which administration of the medicinal solution is completed, the second needle tip 10 side of the needle tube 3 is disposed in the storing recessed portion 36a.

Meanwhile, the plural storing recessed portions 36b are disposed in a suitably spaced relationship from each other in a circumferential direction of a circle centered at the storing recessed portion 36a. The area and the depth of the storing recessed portions 36b in a direction perpendicular to the axial direction of the vessel main body 21 are not specifically limited.

Also with such a prefilled syringe 31 as described above, gas E accommodated in the medicinal solution storage portion 6 together with the medicinal solution M can be partially or entirely kept in the storing recessed portion 36. Accordingly, the amount of gas to be administrated into the living organism past the second needle tip 10 of the needle tube 3 together with the medicinal solution M can be reduced.

### <3. Third Embodiment>

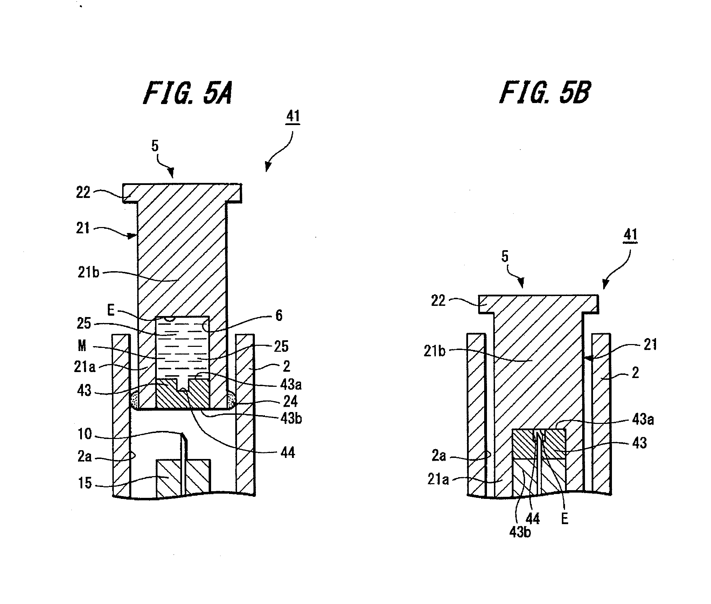

Next, a third embodiment of a prefilled syringe of the present invention is described with reference to FIG. 5.

FIG. 5A is a sectional view showing the prefilled syringe of the third embodiment, and FIG. 5B is a sectional view illustrating a state in which administration of a medicinal solution is completed.

The prefilled syringe 41 according to the present third embodiment is different from the prefilled syringe 1 according to the first embodiment in a medicinal solution vessel and the sealing member. Therefore, description here is given of the medicinal solution vessel and a sealing member, and elements common to the prefilled syringe 1 are denoted by like letters or numerals and overlapping description of the same is omitted. As shown in FIGS. 5A and 5B, the medicinal solution vessel 42 has a vessel main body 21 of a substantially cylindrical shape having a medicinal solution storage portion 6, and a pressing portion 22 provided at one end of the vessel main body 21. A storing recessed portion is not provided on a face of the bottom portion 21 b of the vessel main body 21 on which the medicinal solution storage portion 6 is formed (on the bottom face of the medicinal solution storage portion 6).

Next, the sealing member 43 is described.

The sealing member 43 is slidably inserted in the tubular portion 21 a of the vessel main body 21 and seals the medicinal solution space 25 liquid-tightly. The sealing member 43 has a contact face 43a which can contact in surface contact with the bottom portion 21 b of the vessel main body 21 and an abutting face 43b for abutting with the engaging portion 15 of the needle hub 4.

A storing recessed portion 44 serving as a storing portion is provided on the contact face 43a of the sealing member 43. The storing recessed portion 44 is provided at a position opposing to the second needle tip 10 of the needle tube 3. The cross section of the storing recessed portion 44 in a direction perpendicular to the axial direction of the vessel main body 21 has at least one portion which is formed greater than the outer diameter of the needle tube 3. The second needle tip 10 of the needle tube 3 which penetrates the sealing member 43 is disposed in the storing recessed portion 44.

Also with such a prefilled syringe 41 as described above, gas E accommodated in the medicinal solution storage portion 6 together with the medicinal solution M can be partially or entirely kept in the storing recessed portion 44. Accordingly, the amount of gas to be administrated to the living organism past the second needle tip 10 of the needle tube 3 together with the medicinal solution M can be reduced.

Further, the second needle tip 10 side of the needle tube 3 penetrating the sealing member 43 is disposed in the storing recessed portion 44, and consequently, the second needle tip 10 of the needle tube 3 and the bottom portion 21 b of the medicinal solution vessel 42 do not interfere with each other. As a result, the second needle tip 10 does not scrape the medicinal solution vessel 42, and it can be prevented that a broken piece of the medicinal solution vessel 42 is mixed into the medicinal solution M. Further, it can be prevented that the second needle tip 10 side of the needle tube 3 is deformed to close up the needle hole, and a prescribed amount of the medicinal solution M can be discharged into the living organism from the first needle tip 9 with certainty.

### <4. Fourth Embodiment>

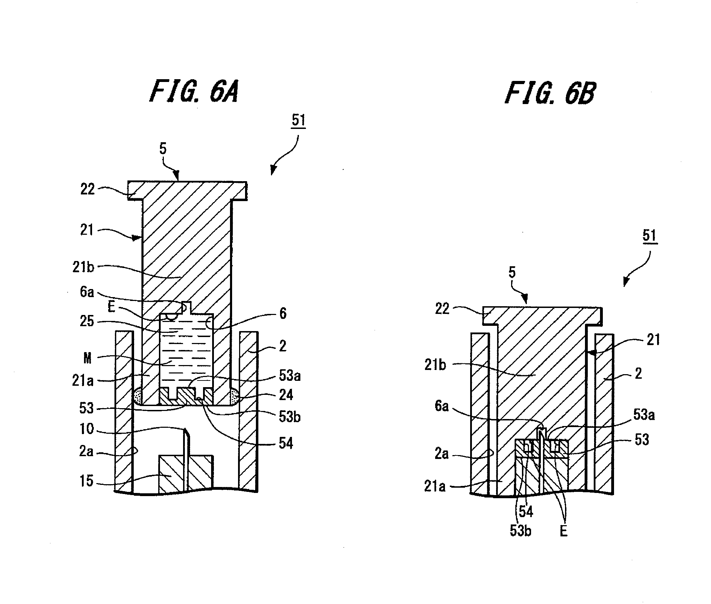

Next, a fourth embodiment of a prefilled syringe of the present invention is described with reference to FIG. 6.

FIG. 6A is a sectional view showing the prefilled syringe of the fourth embodiment, and FIG. 6B is a sectional view illustrating a state in which administration of medicinal solution is completed.

The prefilled syringe 51 according to the fourth embodiment has a configuration similar to that of the prefilled syringe 1 according to the first embodiment. The prefilled syringe 51 is different from the prefilled syringe 1 in that the storing recessed portion is provided also on the sealing member. Therefore, description here is given of the sealing member, and elements common to those of the prefilled syringe 1 are denoted by like letters or numerals and overlapping description of them is omitted.

As shown in FIGS. 6A and 6B, the sealing member 53 is slidably inserted in the tubular portion 21 a of the vessel main body 21 and seals the medicinal solution space 25 liquid-tightly. The sealing member 53 has a contact face 53a which can contact in surface contact with the bottom portion 21 b of the vessel main body 21 and an abutting face 53b for abutting with the engaging portion 15 of the needle hub 4.

A plurality of storing recessed portions 54 serving as storage portions are provided on the contact face 53a of the sealing member 53. The plural storing recessed portions 54 are disposed in a suitably spaced relationship from each other in a circumferential direction of a circle centered at a central portion of the contact face 53a. In particular, the storing recessed portions 54 are provided at a position at which they do not oppose to the second needle tip 10 of the needle tube 3.

When the abutting face 53b of the sealing member 53 is brought into abutment with the engaging portion 15 of the needle hub 4, the second needle tip 10 side of the needle tube 3 protrudes from the contact face 53a of the sealing member 53 (refer to FIG. 6B). Then, in a state in which administration of the medicinal solution is completed, the second needle tip 10 side of the needle tube 3 is disposed in the storing recessed portion 6a.

Also with such a prefilled syringe 51 as described above, gas E accommodated in the medicinal solution storage portion 6 together with the medicinal solution M can be partially or entirely kept in the storing recessed portion 6a and the plural storing recessed portions 54. Accordingly, the amount of gas to be administrated into the living organism past the second needle tip 10 of the needle tube 3 together with the medicinal solution M can be reduced.

It is to be noted that the storing recessed portion provided in the sealing member according to the present invention may be a combination of those of the third embodiment and the fourth embodiment. In other words, on the contact face of the sealing member, a plurality of storing recessed portions may be provided at a central portion and peripheral portions around the central portion. In this instance, in the storing recessed portion provided at the central portion, the second needle tip 10 side of the needle tube 3 penetrating the sealing member is disposed.

### <5. Fifth Embodiment>

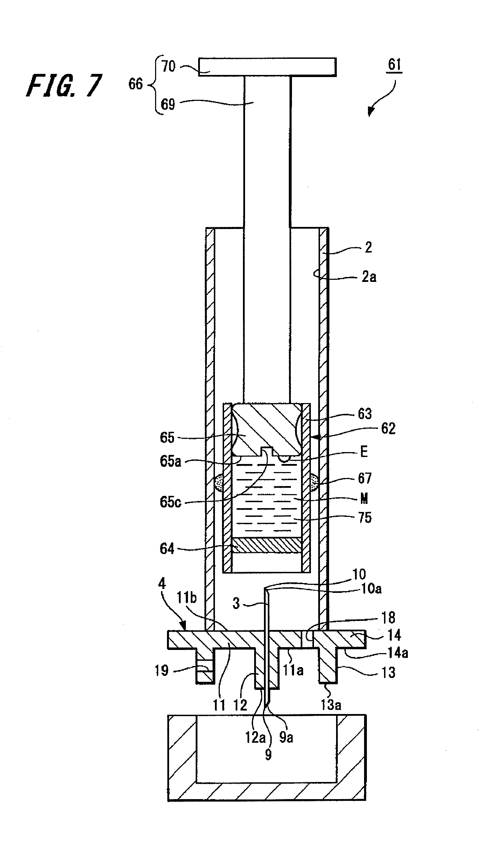

Next, a fifth embodiment of a prefilled syringe of the present invention is described with reference to FIGS. 7 to 9.

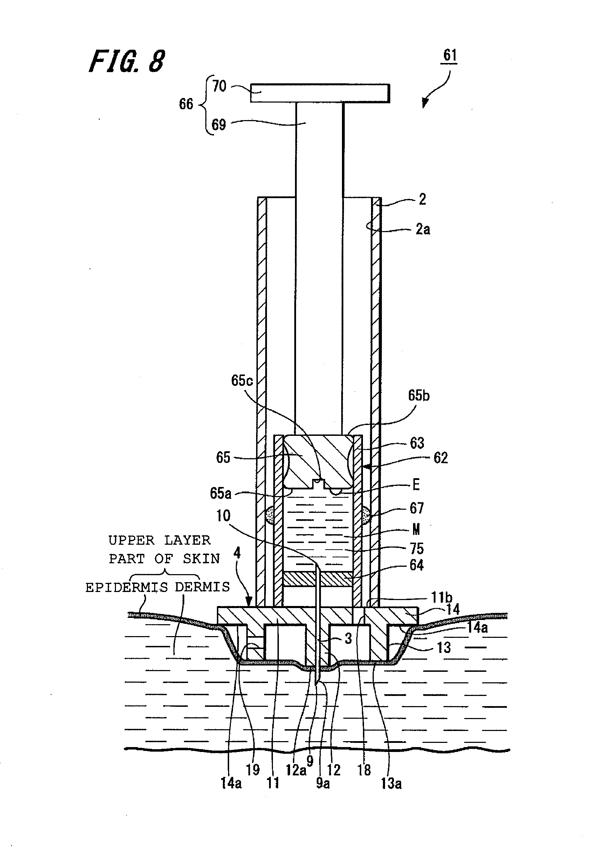

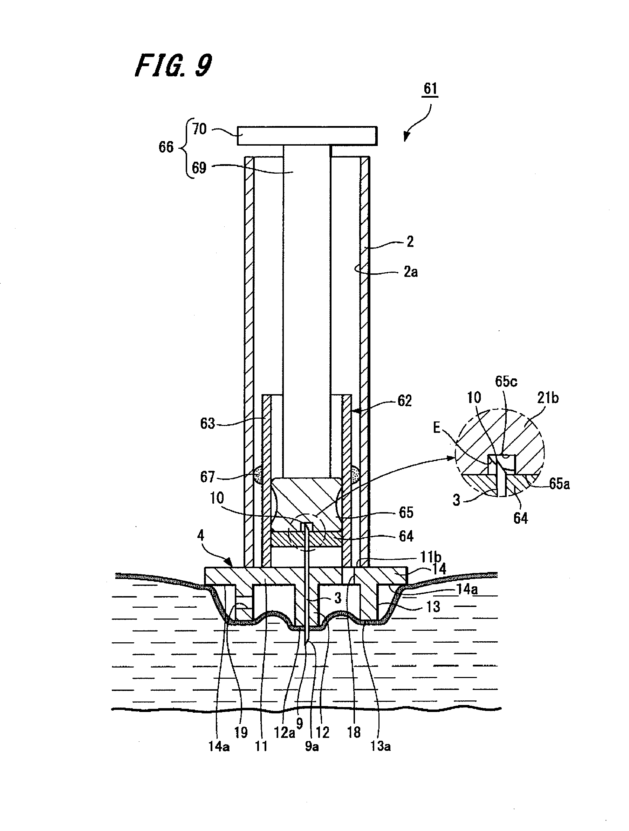

FIG. 7 is a sectional view showing the prefilled syringe of the fifth embodiment, and FIG. 8 is a sectional view illustrating a state in which a needle tube of the prefilled syringe of the fifth embodiment punctures the skin. FIG. 9 is a sectional view illustrating a state in which administration of a medicinal solution is completed.

he prefilled syringe 61 according to the present fifth embodiment is different from the prefilled syringe 1 according to the first embodiment in that a medicinal solution vessel and a pusher are configured as separate members from each other and that the engaging portion 15 is eliminated from the needle hub 4. Therefore, description here is given of the medicinal solution vessel and the pusher, and elements common to the prefilled syringe 61 are denoted by like letters or numerals and overlapping description of the same is omitted.

As shown in FIG. 7, the medicinal solution vessel 62 is inserted in the cylindrical hole 2a from the opening on the other end side of the outer tube 2. The medicinal solution vessel 62 is configured from a tubular portion 63 of a substantially cylindrical shape and a bottom portion 64. The medicinal solution vessel 62 is pushed in by the pusher 66 hereinafter described and thereby slidably moves in the cylindrical hole 2a along the axial direction of the cylindrical hole 2a.

The tubular portion 63 is open at the opposite ends in the axial direction thereof. A seal member 67 is provided on an outer circumferential face of the tubular portion 63. When the medicinal solution vessel 62 is inserted into the cylindrical hole 2a of the outer tube 2, the seal member 67 is interposed between an outer circumferential face of the tubular portion 63 and an inner circumferential face of the outer tube 2. An internal space 20 defined by the cylindrical hole 2a and the hub main body 11 of the needle hub 4 is sealed airtight by the seal member 67. Further, since the seal member 67 closely contacts with the outer circumferential face of the tubular portion 63 and the inner circumferential face of the outer tube 2, the medicinal solution vessel 62 can be held stably at an intermediate portion of the cylindrical hole 2a.

The bottom portion 64 is disposed in such a manner as to close up the (one) opening on one end side in the axial direction of the tubular portion 63 and is fixed to the inner circumferential face of the tubular portion 63 by such a fixing method as a bonding agent. When the medicinal solution vessel 62 is inserted into the cylindrical hole 2a of the outer tube 2, the bottom portion 64 is opposed to the second needle tip 10 of the needle tube 3 held by the needle hub 4. When the prefilled syringe 61 is to be used, the second needle tip 10 side of the needle tube 3 penetrates the bottom portion 64.

The opening on the other end side in the axial direction of the tubular portion 63 is closed up with a sealing member 65. This sealing member 65 has a contact face 65a which can contact in surface contact with the bottom portion 64 of the medicinal solution vessel 62, and an attaching face 65b to which a pusher 66 hereinafter described is attached. It is to be noted that, as an attachment method of the pusher 66, screwing, fitting and so forth are applicable. The sealing member 65 is slidably inserted in the tubular portion 63 of the medicinal solution vessel 62 and seals a medicinal solution space 75 liquid-tightly. The medicinal solution space 75 is an enclosed sealed airtight space by the sealing member 65 and maintains a sterilized state.

Further, a storing recessed portion 65c is provided on the contact face 65a of the sealing member 65. The storing recessed portion 65c is provided at a position opposing to the second needle tip 10 of the needle tube 3. The cross section of the storing recessed portion 65c in a direction perpendicular to the axial direction of the sealing member 65 has at least one portion which is formed greater than the outer diameter of the needle tube 3. Further, the depth of the storing recessed portion 65c is set equal to or greater than the length by which the second needle tip 10 side of the needle tube 3 protrudes from the bottom portion 64. In a state in which administration of the medicinal solution M is completed, the second needle tip 10 side of the needle tube 3 protruding from the bottom portion 64 is inserted in the storing recessed portion 65c.

It is to be noted that, although the material of the sealing member 65 and the bottom portion 64 of the medicinal solution vessel 62 is not limited specifically similarly to the sealing member 23 in the first embodiment, preferably it is configured from an elastic material in order to assure good liquid-tightness with the medicinal solution storage portion 6.

Next, the pusher 66 is described.

The pusher 66 has a plunger 69 formed from a bar-like member, and an operating portion 70 of a substantially disk-like shape. The plunger 69 is attached at one end portion in the axial direction thereof to the sealing member 65. The operating portion 70 is provided at the other end of the plunger 69 in the axial direction.

Further, as shown in FIG. 8, if the user pushes the operating portion 70, then the sealing member 65 and the medicinal solution vessel 62 are pushed in in the axial direction of the cylindrical hole 2a of the outer tube 2. Then, the tubular member 63 of the medicinal solution vessel 62 contacts at one end thereof with the other end face 11 b of the hub main body 11. At this time, the second needle tip 10 of the needle tube 3 punctures the bottom portion 64 of the medicinal solution vessel 62 and penetrates the bottom portion 64. Consequently, liquid communication to the needle tube 3 is carried out.

Further, if the operating portion 70 is pushed after the second needle tip 10 punctures into the medicinal solution vessel 62 and then the liquid communication into the needle tube 3 is completed, then the sealing member 65 is pushed in to the bottom portion 64 side as illustrated in FIG. 9. Since the sealing member 65 is slidably moved along the axial direction in the tubular member 63, the medicinal solution M in the medicinal solution vessel 62 is discharged into the living organism from the first needle tip 9 past the second needle tip 10 of the needle tube 3.

Thereafter, when the contact face 65a of the sealing member 65 is brought into surface contact with the bottom portion 64 of the medicinal solution vessel 62, the administration of the medicinal solution M using the prefilled syringe 61 of the present embodiment is completed. At this time, even if gas E is accommodated in the medicinal solution storage portion 6 together with the medicinal solution M, the gas E can be partially or entirely kept in the storing recessed portion 65c. Accordingly, the amount of gas to be administrated into the living organism past the second needle tip 10 of the needle tube 3 together with the medicinal solution M can be reduced.

Further, the second needle tip 10 side of the needle tube 3 penetrating the bottom portion 64 is inserted into the storing recessed portion 65c. In other words, the storing recessed portion 65c has a function also as a cutaway portion for avoiding interference between the second needle tip 10 side of the needle tube 3 and the bottom portion 64 of the medicinal solution vessel 62. As a result, it can be prevented that the needle hole of the second needle tip 10 side is closed up with the sealing member 65, and a prescribed amount of the medicinal solution M can be discharged into the living organism from the first needle tip 9 with certainty.

While the fifth embodiment described above is configured such that the storing recessed portion 65c is provided on the sealing member 65, also it is possible to use another configuration wherein a storing recessed portion is provided on the bottom portion 64 of the medicinal solution vessel 62. Further, in the fifth embodiment described above, the bottom portion 64 of the medicinal solution vessel 62 is entirely formed from an elastic member. However, only it is necessary for the bottom portion in the fifth embodiment to be able to be penetrated by the needle tube 3, and an elastic member may be used at least for the portion which is penetrated by the needle tube 3.

Further, while, in the present embodiment, the engaging portion 15 is eliminated from the needle hub 4, the engaging portion 15 may be provided. In this instance, when the sealing member 65 and the medicinal solution vessel 62 are pushed in in the axial direction of the cylindrical hole 2a of the outer tube 2, the bottom portion 64 of the medicinal solution vessel 62 is brought into abutment with the engaging portion 15.

The present invention is not limited to the embodiments described hereinabove and shown in the drawings but can be carried out in various modified forms without departing from the subject matter of the present invention described in the claims. For example, in the embodiments described hereinabove, communication of liquid into the needle tube is carried out by causing the needle tube to penetrate the sealing member or the bottom portion of the medicinal solution vessel. However, the prefilled syringe of the present invention may be configured such that communication of liquid into the needle tube held by the needle hub is carried out by mounting the needle hub on the medicinal solution vessel.

Further, the embodiments described above are configured such that the storing recessed portion is provided at one portion of one of the sealing member and the bottom portion of the medicinal solution vessel. However, in the prefilled syringe of the present invention, the storing recessed portion may be provided on both of the sealing member and the bottom portion of the medicinal solution vessel. In this instance, both of the storing recessed portions may be opposed to each other or may not be opposed to each other. Further, an example wherein an adjustment portion, a stabilization portion and a guide portion which is a pressure indication portion are provided on the needle hub in order to administrate a medicinal solution to the upper layer part of the skin is described. However, the needle hub is not limited to this, but the adjustment portion, stabilization portion and guide portion may not be provided on the needle hub. Further, the prefilled syringe of the present invention can be applied also to a prefilled syringe for subcutaneously feeding medicinal solution. In particular, by elongating the length (protruding length L) of the first needle tip 9 side of the needle tube 3 which protrudes from the skin protruding face 12a, a medicinal solution can be subcutaneously fed to a portion of the skin deeper than the upper layer part of the skin. Further, the size (gauge) of the needle tube can be suitably selected in response to an application.

### EXPLANATIONS OF LETTERS OR NUMERALS

1, 31, 41, 51, 61: Prefilled syringe, 2: Outer tube, 2a: Cylindrical hole, 3: Needle tube, 4: Needle hub, 5, 35, 42, 62: Medicinal solution vessel, 6: Medicinal solution storage portion, 6a, 36, 44, 54, 65c: Storing recessed portion (storage portion), 7: Cap, 9: First needle tip, 10: Second needle tip, 11: Hub main body, 12: Adjustment portion, 13: Stabilization portion, 14: Guide portion, 15: Engaging portion, 18: First vent hole, 19: Second vent hole, 20: Internal space, 21: Vessel main body, 21 a, 63: Tubular portion, 21 b, 64: Bottom portion, 22: Pushing portion, 23, 43, 53, 65: Sealing member, 23a, 43a, 53a, 65a: Contact face, 23b, 43b, 53b: Abutting face, 24, 67: Seal member, 25, 75: Medicinal solution space, 65b: Attaching face, 66: Pusher, 69: Plunger, 70: Operating portion, B: Bevel length, L: Protruding length, S: Distance from a circumferential edge of the needle protruding face to a circumferential face of the needle tube, T: Distance from an inner wall face of the stabilization portion to an outer circumferential face of the adjustment portion, x: Guide portion length, y: Guide portion height, d: Inner diameter

## Claims

1. A prefilled syringe, comprising:
an outer tube open at the opposite ends thereof;
a medicinal solution vessel slidably movable in the outer tube, open at one end thereof and having a bottom portion at the other end thereof;
medicinal solution filled in the medicinal solution vessel;
a sealing member for sealing the one end of the medicinal solution vessel;
a pushing portion for operating movement of the medicinal solution vessel;
a needle tube provided at one end thereof a first needle tip for puncturing a living organism and provided at the other end thereof a second needle tip for puncturing the bottom portion of the medicinal solution vessel or the sealing member;
a needle hub holding an intermediate portion of the needle tube and connected to one end of the outer tube such that the second needle tip of the needle tube is disposed in the outer tube; and
a seal member for sealing between an inner circumferential face of the outer tube and an outer circumferential face of the medicinal solution vessel;
wherein at least one of the sealing member and the bottom portion of the medicinal solution vessel provides thereon a storing portion for storing gas in the medicinal solution vessel.

2. The prefilled syringe according to claim 1, wherein, when the sealing member moves in the medicinal solution vessel and comes near to the bottom portion, the second needle tip side of the needle tube is disposed into the storing portion.

3. The prefilled syringe according to claim 1, wherein the sealing member has a contact face which can contact in surface contact with the bottom portion.

4. The prefilled syringe according to claim 1, wherein the needle hub has an engaging portion for engaging with the sealing member which moves in a cylindrical hole of the outer tube together with the medicinal solution vessel so that the sealing member slidably moves in the medicinal solution vessel.

5. The prefilled syringe according to claim 1, wherein the storing portion is a recessed portion.

6. The prefilled syringe according to claim 1, wherein, in a state in which administration of the medicinal solution is completed, gas in the medicinal solution vessel is accommodated in the storing portion.
